# EUROPÄISCHE PATENTSCHRIFT

(11) **EP 3 456 242 B1**
(45) Veröffentlichungstag und Bekanntmachung des Hinweises auf die Patenterteilung: **28.10.2020**
(21) Anmeldenummer: 17190821.3
(22) Anmeldetag: 13.09.2017
(51) Int. Cl.: A61B 5/00, A61B 5/055, G01R 33/54

(54) **EINSTELLEN EINER TISCHPOSITION EINES TOMOGRAPHEN**
ADJUSTMENT OF A TABLE POSITION FOR A TOMOGRAPHIC APPARATUS
RÉGLAGE D'UNE POSITION DE TABLE D'UN TOMOGRAPHE

(43) Veröffentlichungstag der Anmeldung: 20.03.2019
(73) Patentinhaber: Siemens Healthcare GmbH, 91052 Erlangen (DE)
(72) Erfinder: Keil, Miriam, 91056 Erlangen-Dechsendorf (DE); Rick, Manuela, 91077 Neunkirchen am Brand (DE)

(56) Entgegenhaltungen:
- DE-A1-102014 201 242
- DE-A1-102015 205 004

## Beschreibung

Die Erfindung betrifft ein Verfahren zum Einstellen einer Tischposition für einen Tisch eines Tomographen zur Messung eines Körperbereichs eines auf dem Tisch befindlichen Untersuchungsobjektes. Zu der Erfindung gehört auch ein entsprechender Magnetresonanztomograph, eine entsprechende Recheneinheit, ein Computerprogramm sowie ein computerlesbarer Datenträger.

Bei dem Untersuchungsobjekt oder auch Patienten handelt es sich meist um einen Menschen bzw. einen menschlichen Körper. Grundsätzlich kann der Patient auch ein Tier sein. Im Folgenden werden daher die beiden Begriffe "Untersuchungsobjekt" und "Patient" synonym verwendet. Das Untersuchungsobjekt kann alternativ eine Pflanze oder ein nicht-lebender Gegenstand aus Metall oder Gestein, z.B. ein historisches Artefakt oder dergleichen sein.

Mit einer Messung oder Untersuchung ist das Gewinnen von Bilddaten eines Körperbereichs des Untersuchungsobjektes mittels des Tomographen gemeint. Eine Untersuchung beginnt damit, dass ein Körper auf den genannten Tisch gelegt wird und daraufhin der Tisch in eine Tischposition verfahren wird oder eine Tischposition eingestellt wird, an welcher der zu untersuchende Körperbereich eine günstige Position bezüglich eines Isozentrums des Tomographen aufweist.

Als Isozentrum wird insbesondere der Raumbereich mit der höchsten Qualität der Abbildungsleistung oder Abbildungstreue des Tomographen bezeichnet. Das Isozentrum kann also beispielsweise das Zentrum des kalibrierten Raumbereichs des Tomographen sein. Standardmäßig wird im Sinne einer bestmöglichen Bildqualität folglich eine Tischposition eingestellt, in welcher der zu untersuchende Körperbereich so dicht wie möglich am Isozentrum, idealerweise genau im Isozentrum, angeordnet ist, eingestellt. Diese Tischposition wird im Folgenden und im Sinne der Erfindung als Isozentrumstischposition bezeichnet.

Nach der Positionierung des Untersuchungsobjektes im Tomographen erfolgt die eigentliche Untersuchung, sprich, die Erzeugung von Tomographieaufnahmen oder Bilddaten auf an sich bekannte Weise.

Nachdem die gewünschten Bilddaten von dem Körperbereich und unter Umständen weiterer Körperbereiche gewonnen worden sind, kann der Körper wieder von dem Tisch entfernt werden, also beispielsweise der Patient den Tisch verlassen. Damit ist die Untersuchung beendet.

Die Untersuchung kann von einem Benutzer, z.B. einem Arzt oder Assistenten (MTA - medizinisch-technischer Assistent) geleitet oder überwacht werden. Zu dem auf dem Tisch angeordneten Untersuchungsobjekt kann der Benutzer zunächst ein Untersuchungsprogramm auswählen, die Auswahl kann von einer medizinischen Fragestellung oder einem bekannten Krankheitsbild abhängen. Ein Untersuchungsprogramm kann in Untersuchungsschritte unterteilt sein. Bei aktiviertem Untersuchungsprogramm kann eine Steuereinheit des Tomographen den Benutzer beispielsweise auffordern, für einen ersten Untersuchungsschritt mittels Lasermarker des Tomographen z.B. einen Körperteil oder Körperbereich oder eine Position am Körper des Patienten, z.B. die Leber oder das Herz, auszuwählen oder zu markieren. Über diese Interaktion kann der Benutzer festlegen, zu welcher Position am Patientenkörper der Benutzer die Erzeugung von Bilddaten wünscht, die dann während der Messung idealerweise im Isozentrum des Tomographen angeordnet wird. Im Folgenden wird diesbezüglich von der Isozentrumsposition, also der durch den Benutzer markierten, abzubildenden Position am Körper des Patienten gesprochen.

Der Lasermarker ist bspw. am Tomographengehäuse oberhalb der Spulenöffnung angeordnet und sendet einen Laserstrahl vertikal nach unten aus. Für die Markierung wird der Tisch mit darauf befindlichem Patienten derart verschoben, dass die interessierende Körperposition im Laserstrahl zu liegen kommt. Da die Relativlage von Lasermarker bzw. Laserstrahl zu Isozentrum des Tomographen vorab festgelegt bzw. bekannt ist, lässt sich nun auch die Isozentrumstischposition ableiten und einstellen. Es kann vorgesehen sein, dass mit eingestellter Isozentrumstischposition eine Übersichtsaufnahme, ein sogenanntes Localizer-Bild, erzeugt wird, welches bei einer anschließenden Protokollplanung zum Einsatz kommen kann und ggf., um die durch den Benutzer definierte Isozentrumstischposition zu überprüfen.

In einem zweiten Untersuchungsschritt kann vorgesehen sein, dass der Benutzer eine Fokusposition, also eine zweite, insbesondere von der Isozentrumsposition abweichende Körperposition in dem Localizer-Bild des Patienten markiert bzw. auswählt. So kann auch die Fokus-Position bspw. über einem bestimmten Organ oder einer speziellen anatomischen Struktur liegen, die der Benutzer mit der Messung zentral abbilden möchte. Dies ist dann von Vorteil, wenn sich die Wahl der Isozentrumsposition in der Übersichtsaufnahme als ungenau oder falsch erweist oder einer der folgenden Messschritte nicht die maximale Bildqualität erfordert. Dann kann eine Messung auch außerhalb der Isozentrumsposition, bspw. der Fokusposition, erfolgen. Dies spart bzw. minimiert vorteilhaft Tischbewegungen und sorgt für einen höheren Komfort bei der Messung. Da die Relativlage von Fokusposition und Isozentrumsposition bekannt ist, kann auch auf eine entsprechende Fokustischposition geschlossen werden, in welcher der Tisch so eingestellt ist, dass der darauf befindliche Patient mit der Fokusposition im Isozentrum des Tomographen zu liegen kommt. Die Auswahl der bei einer Messung einzustellenden Tischposition wird dem Benutzer im Rahmen des sogenannten Scan@Center-Konzeptes bereit gerstellt.

In einem anderen Untersuchungsschritt kann der Benutzer ein Messprotokoll auswählen, mittels welchem Bilddaten er-zeugt werden sollen. Ein Messprotokoll ist Menge aller Parameter, die bei dem Tomographen eingestellt werden müssen, um für den Körperbereich die benötigten Bilddaten zu erzeugen. Der Benutzer aktiviert dann die Aufnahme, das heißt das ausgewählte Messprotokoll wird ausgeführt. Hierdurch entstehen die Bilddaten von der ausgewählten oder markierten Körperregion. Danach kann in einem nächsten Untersuchungsschritt das Gewinnen von Bilddaten eines anderen Körperbereichs bzw. von Bilddaten desselben Körperbereichs umfassend andere Bildinformation, bspw. einen anderen Kontrast, etc., vorgesehen sein. Sind alle Untersuchungsschritte, jeweils unter Verwendung eines dafür vorgesehenen Messprotokolls abgearbeitet, so ist das Untersuchungsprogramm beendet. Damit ist die Untersuchung beendet. Die Folge der Untersuchungsschritte stellt einen Arbeitsablauf oder Work-Flow für den Benutzer dar.

Indem alle für das Gewinnen von Bilddaten eines Körperbereichs benötigten Parameter des Tomographen zu einem Messprotokoll zusammengefasst sind, kann sichergestellt werden, dass die beste bzw. eine erforderliche Bildqualität erlangt wird, ohne dass sich der Benutzer um technische Details kümmern muss.

Bei der Planung jedes einzelnen Untersuchungsschrittes, auch Protokoll- oder Schichtplanung genannt, kann der Benutzer bspw. anhand des oben erwähnten Localizer-Bildes oder einer vergleichbaren Aufnahme eine Fein-Justage der Isozentrums- bzw. der Fokusposition vornehmen, also der Position im Körperbereich, welche für die Messung im Iso-zentrum angeordnet werden soll.

Bislang wird dem Benutzer dazu in dem Localizer-Bild ein abzubildender Auswahlbereich entsprechend einem Field of View des Tomographen bzw. entsprechend einer geplanten Schichtgruppe immer auf die Isozentrumsposition ausgerichtet angezeigt. Mit anderen Worten wird ein Zentrum der geplanten Schichtgruppe der Isozentrumsposition überlagert bzw. darauf zentriert angezeigt. Dies geschieht standardmäßig unabhängig davon, ob der Benutzer initial eine gewünschte Fokusposition eingegeben hat oder nicht. Mit anderen Worten bleibt eine Angabe über eine Fokusposition bei der Schichtplanung unberücksichtigt. Dies hat zur Folge, dass ggf. die Planung jedes einzelnen Untersuchungsschrittes nicht nur eine aufwändige manuelle Anpassung des Auswahlbereichs bzw. des Schichtgruppen-Zentrums an die Fokusposition erfordert, sondern auch mehr und ggf. längere Verstellschritte notwendig sind, um die für die Messung ausgewählte Tischposition zu erreichen. Dadurch Verlängern sich Vorbereitungszeiten und der Patientenkomfort sinkt. Zudem verlängern sich die Untersuchungszeiten, wodurch die Anzahl untersuchter Patienten pro Tag beschränkt ist.

DE 10 2015 205 004 A1 betrifft das Einstellen einer Tischposition eines Tomographen, wobei Bilddaten von einem Körperbereich mittels des Tomographen durch Ausführen eines Messprotokolls erzeugt werden. Das Messprotokoll soll unabhängig vom abgebildeten Körperbereich verwendbar sein. Dabei werden ein Iso-Modus, in welchem die Tischposition eine Relativlage des Körperbereichs bezüglich eines Isozentrums des Tomographens gemäß einem Optimierungskriterium eingestellt wird, sowie ein Ref-Modus, in welchem die Tischposition auf eine vorgegebene Referenzposition eingestellt wird, bereitgestellt, wobei die Auswahl zwischen dem Iso-Modus und dem Ref-Modus automatisch in Abhängigkeit von einer anatomischen Information des Körperbereichs und/oder einer sich in der Referenzposition ergebenden Relativlage des Körperbereichs bezüglich des IsoZentrums ausgewählt und eingestellt wird. Dort ist die Fragestellung, ob der Körperbereich abseits vom Isozentrum noch hinreichend genau vermessen werden kann. Um dies zu überprüfen, wird insbesondere ein Nahe-Genug-Kriterium verwendet.

DE 10 2014 201 242 A1 offenbart ein Verfahren zur Bestimmung einer geeigneten Tischposition für einen MR-Untersuchungsschritt, wobei der Patiententisch an eine erste Tischposition bewegt wird und ein erstes Messvolumen ausgewählt wird. Dann werden Justagemessungen zur Einstellung von Komponenten des MR-Gerätes auf das erste Messvolumen durchgeführt und die Messergebnisse gespeichert. Sodann erfolgt die Auswahl eines zweiten Messvolumens und das Berechnen der Differenz zwischen einer optimalen Tischposition für den zweiten Untersuchungsschritt und der ersten Tischposition, wobei bei der Aufnahme der MR-Daten von dem zweiten Messvolumen die optimale Tischposition für den zweiten Untersuchungsschritt verwendet wird, sobald die Differenz einen bestimmten Schwellwert überschreitet.

Der Erfindung liegt daher die Aufgabe zugrunde, alternative Mittel bereit zu stellen, die es erlauben, das Einstellen einer Tischposition zu vereinfachen, zu beschleunigen und einen höheren Komfort für einen Patienten bei einer Messung zu erzielen.

Diese Aufgabe wird durch die Gegenstände der unabhängigen Patentansprüche gelöst. Bevorzugte und/oder alternative, vorteilhafte Ausgestaltungsvarianten sind Gegenstand der abhängigen Ansprüche.

Nachstehend wird die erfindungsgemäße Lösung der Aufgabe in Bezug auf das beanspruchte Verfahren als auch in Bezug auf die beanspruchten Vorrichtungen beschrieben. Hierbei erwähnte Merkmale, Vorteile oder alternative Ausführungsformen sind ebenso auch auf die anderen beanspruchten Gegenstände zu übertragen und umgekehrt. Mit anderen Worten können gegenständliche Ansprüche (die beispielsweise auf ein Verfahren gerichtet sind) auch mit Merkmalen, die in Zusammenhang mit einer der Vorrichtungen beschrieben oder beansprucht sind, weitergebildet sein. Die entsprechenden funktionalen Merkmale des Verfahrens werden dabei durch entsprechende gegenständliche Module oder Einheiten ausgebildet.

Die vorliegende Erfindung betrifft in einem ersten Aspekt ein Verfahren zum Einstellen einer Tischposition für einen Tisch eines Tomographen zur Messung eines Körperbereichs eines auf dem Tisch befindlichen Untersuchungsobjektes. Das Verfahren umfasst eine Vielzahl von Schritten.

Ein erster Schritt umfasst ein an sich bekanntes Erfassen einer Benutzerauswahl eines Messprotokolls aus einer Mehrzahl möglicher Messprotokolle. Die Mehrzahl der Messprotokolle bildet zusammen ein Messprogramm. Das Erfassen kann ein Empfangen bzw. ein Erhalten einer Benutzereingabe oder Benutzerauswahl sein, welche über eine Ausgabe- und Eingabeeinheit des Tomographen erfolgen kann, umfassen.

Ein zweiter Schritt umfasst ein an sich bekanntes Erfassen bzw. Empfangen einer Benutzereingabe bezüglich einer Isozentrumsposition und ein dritter Schritt umfasst ein an sich bekanntes Erfassen bzw. Empfangen einer Benutzereingabe bezüglich einer Fokusposition, wie eingangs bereits beschrieben. Nochmals sei erwähnt, dass die Isozentrumsposition bspw. mittels Lasermarker festgelegt werden kann. In diesem Sinne umfasst das Erfassen auch die manuelle Positionierung des Patienten unter dem Laserstrahl durch den Benutzer. Die Fokusposition kann bspw. am Anfang einer Messung in einem Localizer-Bild markiert werden. Die Fokusposition kennzeichnet bspw. ein Organ oder eine bestimmte anatomische Struktur. Die Fokusposition definiert, wo relativ zur Anatomie des Patienten das Schichtzentrum eines Messschrittes angeordnet sein soll und legt somit auch die Fokustischposition fest, in die der Tisch für die Messung verfahren werden muss. Die Fokusposition weicht in der Regel von der Isozentrumsposition ab.

In einem vierten Schritt erfolgt ein Anzeigen einer Übersichtsdarstellung des zu untersuchenden Körperbereichs umfassend einen Auswahlbereich und einen Indikator der Fokusposition relativ zu dem Körperbereich, wobei der Auswahlbereich auf die Fokusposition ausgerichtet an-gezeigt wird. Bei der Übersichtsdarstellung kann es sich bspw. um das Localizer-Bild oder eine vergleichbare Abbildung des zu untersuchenden Körperbereichs handeln. Die Anzeige erfolgt für den Benutzer, bspw. bevorzugt über eine Ausgabeeinheit des Tomographen. Die Anzeige kann aber auch auf eine entfernte Ausgabeeinheit, bspw. ein Monitor, übertragen und dort angezeigt werden, die bspw. in einem Nebenraum angeordnet ist. Die Ausgabeeinheit kann bspw. auch als portable Ausgabeeinheit, bspw. ein Tablet oder ausgebildet sein, sodass sich der Benutzer während der Protokollplanung bewegen kann.

Der Auswahlbereich repräsentiert einen Teil des zu untersuchenden Körperbereichs, welcher durch die per Messprotokoll vorgegebene Untersuchung abgebildet wird. Der Auswahlbereich kann einem Field of View des Tomographen entsprechen. Erfindungsgemäß kann die Anzeige des Auswahlbereichs auch eine Anzeige der geplanten Schichtgruppe, also eine Anzeige der Lage, Breite, Anzahl, Verteilung, etc. der mit der Messung untersuchten Schichten umfassen. Insbesondere kann erfindungsgemäß eine Anzeige des Schichtgruppenzentrums umfasst sein.

Der Indikator der Fokusposition entspricht einer optischen Markierung der durch den Benutzer anfänglich festgelegten Fokusposition relativ zur der abgebildeten Anatomie im zu untersuchenden Körperbereich, bspw. in Form einer beliebig ausgebildeten Linie, aber auch in Form eines am Rand der Übersichtsdarstellung angeordneten Dreiecks, wobei eine Spitze des Dreiecks die Fokusposition markiert.

Die Anzeige kann auch einen weiteren Indikator zur Veranschaulichung der Lage der Isozentrumsposition umfassen, bspw. ebenfalls in Form einer Linie.

Die Erfinder haben also erkannt, dass es entgegen der gängigen Praxis vorteilhaft sein kann, bei der Protokollplanung die Fokusposition anstelle der Isozentrumsposition als initiale Anzeigeposition für den Auswahlbereich zu verwenden. Mit anderen Worten wird erfindungsgemäß beim Anzeigen der Übersichtsdarstellung der Auswahlbereich so angeordnet, dass das Zentrum des Auswahlbereichs bzw. ein entsprechendes Schichtgruppenzentrum und die Fokusposition überlagert sind. Mit anderen Worten wird der Auswahlbereich auf die Fokusposition zentriert angezeigt.

In Schritt fünf des erfindungsgemäßen Verfahrens erfolgt ein Erfassen einer Benutzereigabe bezüglich einer veränderten Fokusposition. Die veränderte Fokusposition kann der initial ausgewählten Fokusposition entsprechen, aber auch von dieser abweichen. Die Benutzereingabe kann folglich in Form einer Bestätigung der Fokusposition oder in Form einer Korrektur der Fokusposition erfolgen. In einem sechsten Verfahrensschritt erfolgt ein Ausrichten des Auswahlbereichs auf die veränderte Fokusposition. Bevorzugt erfolgen Schritt fünf und sechs zusammen, indem bspw. der Benutzer über die Ausgabe- und Eingabeeinheit des Tomographen in der Übersichtsdarstellung den Auswahlbereich bzw. das Zentrum des Auswahlbereichs oder das Schichtzentrum auf die gewünschte, veränderte Fokusposition verschiebt bzw. die Fokusposition bestätigt. In letzterem Fall umfasst das Ausrichten des Auswahlbereichs die Beibehaltung seiner Position. Alternativ kann der Benutzer eine veränderte Fokusposition über die Eingabe von Koordinaten bezüglich eines Referenzkoordinatensystems des Tomographen mittels Eingabeeinheit auswählen und der Auswahlbereich automatisch in der Übersichtdarstellung dorthin verschoben.

In Schritt sieben erfolgt ein Einstellen der Tischposition auf eine Fokustischposition entsprechend der Fokusposition, sofern, die veränderte Fokusposition ein Prüfkriterium erfüllt und Einstellen auf eine Isozentrumstischposition entsprechend der Isozentrumsposition, sofern die veränderte Fokusposition das Prüfkriterium verletzt. Dieser Schritt umfasst also eine Prüfung der veränderten Fokusposition im Hinblick auf ein Prüfkriterium. Das Prüfkriterium ist vorab festgelegt und durch den Benutzer in aller Regel nicht veränderbar. Ergibt die Prüfung der veränderten Fokusposition ein positives Ergebnis, sprich, ist das Kriterium erfüllt, wird der Tisch in die Fokustischposition verfahren bzw. gebracht, bzw. verbleibt dort, wenn der Tisch sich für einen vorherigen Untersuchungsschritt schon in der Fokustischposition befindet. Nur für die Fälle, in denen die Prüfung ein negatives Ergebnis liefert, sprich, das Prüfkriterium verletzt ist, wird der Tisch in die Isozentrumstischposition verbracht. Das Prüfkriterium selbst ist systemspezifisch und durch den Benutzer nur bei entsprechender Qualitfikation, Berechtigung und/oder Erfahrung editierbar. nicht editierbar.

Die Erfinder haben also erkannt, dass es entgegen der gängigen Praxis vorteilhaft sein kann, bei der Protokollplanung die Fokusposition anstelle der Isozentrumsposition als initiale Anzeigeposition für den Auswahlbereich zu verwenden. Mit anderen Worten wird erfindungsgemäß beim Anzeigen der Übersichtsdarstellung der Auswahlbereich so angeordnet, dass das Zentrum des Auswahlbereichs bzw. ein entsprechendes Schichtgruppenzentrum und die Fokusposition überlagert sind. Mit anderen Worten wird der Auswahlbereich auf die Fokusposition zentriert angezeigt. In einer Vielzahl von Anwendungsfällen wird die gewünschte Fokusposition mit der initialen Fokusposition übereinstimmen oder nur minimal davon abweichen. Erfindungsgemäß wird also der Planungsaufwand reduziert, da nun keine oder nur noch minimale Anpassungen des Auswahlbereichs für die Messung vorgenommen werden brauchen. Die Protokollplanung beschleunigt sich und Tischbewegungen vor einer Messung werden reduziert oder sogar überflüssig.

In einem Ausführungsbeispiel der Erfindung berücksichtigt das Prüfkriterium ein Abstandsmaß zwischen der veränderten Fokusposition und der Isozentrumsposition und ist erfüllt, sofern das Abstandmaß ein vorab festgelegtes Höchstmaß nicht überschreitet. Anders ausgedrückt berücksichtigt das Prüfkriterium eine Relativlage zwischen zu untersuchendem Körperbereich, insbesondere der darin definierten, veränderten Fokusposition zum Isozentrum des Tomographen, wenn eine Tischposition entsprechend der veränderten Fokusposition, also eine zweite Fokustischposition eingestellt würde. Anders ausgedrückt berücksichtigt das Prüfkriterium eine Relativlage zwischen geplantem Schichtzentrum und Isozentrum des Tomographen. Nur wenn dieser Abstand ein Höchstmaß einhält, wird für die Messung als Tischposition die Fokustischposition eingestellt. In einem anderen Ausführungsbeispiel der Erfindung berücksichtigt das Prüfkriterium ein Abstandsmaß zwischen einem von der Isozentrumsposition am weitesten entfernten Punkt des Auswahlbereichs und der Isozentrumsposition und ist erfüllt, sofern das Abstandmaß ein vorab festgelegtes Höchstmaß nicht überschreitet. In dieses Abstandsmaß fließt Information über die Ausdehnung der Schichtgruppe ein. Mit anderen Worten wird betrachtet, wie groß der Abstand zwischen dem Isozentrum und einem durch eine Messung gerade noch abgebildeten Körperteil des zu untersuchenden Körperbereichs ist, wenn eine Tischposition entsprechend der veränderten Fokusposition, also eine zweite Fokustischposition eingestellt würde. Wenn dieser Abstand ein Höchstmaß einhält, wird für die Messung als Tischposition die Fokustischposition eingestellt.

In einem weiteren Ausführungsbeispiel der Erfindung ist das vorab festgelegte Höchstmaß abhängig von einer anatomischen Information und/oder einer Information bezüglich der Homogenität eines Magnetfeldes des Tomographen. Hierdurch kann einerseits berücksichtigt werden, dass einige Körperbereiche auch in einem größeren Abstand zum Isozentrum noch mit einer vorbestimmten Mindestbildqualität abgebildet werden können als es bei anderen Körperbereichen der Fall sein kann. Andererseits wird so berücksichtigt, in welchem Abstand ausgehend vom Isozentrum noch mit einer vorgegebenen Mindestqualität abgebildet werden kann. Dies wird durch die Homogenität des magnetischen Feldes bestimmt oder beeinflusst. In vorteilhafter Weise wird das Höchstmaß so an die Anatomie und/oder die Homogenität des Magnetfeldes bzw. Abbildungsleistung des Tomographen angepasst.

In einem weiteren Ausführungsbeispiel erfolgt das Anzeigen der Übersichtsdarstellung des zu untersuchenden Körperbereichs mit auf die Fokusposition ausgerichtetem Auswahlbereich, sofern ein für das ausgewählte Messprotokoll eingestellter Betriebsmodus dies vorsieht. Mit anderen Worten wird erfindungsgemäß ein Betriebsmodus berücksichtigt. Wurde hingegen ein Messprotokoll ausgewählt, dessen Betriebsmodus eine erfindungsgemäße Anzeige des Auswahlbereichs verbietet, kann bspw. eine Anzeige erfolgen, bei der der Auswahlbereich auf die Isozentrumsposition ausgerichtet ist.

Ein Betriebsmodus definiert, ob ein Messprotokoll bzw. ein Messschritt unter Einhaltung der Isozentrumstischposition durchgeführt werden muss oder ob davon abweichende Tischpositionen, bspw. die Fokustischposition oder andere Tischpositionen, eingenommen werden können. Jedes Messprogramm umfassend eine Vielzahl von Messprotokollen umfasst auch eine Angabe in Form eines für alle umfassten Messprotokolle geltenden, globalen Positionsparameters. Der globale Positionsparameter definiert also einen Betriebsmodus für alle Messprotokolle. Die Einstellung des globalen Positionierungsparameters erfolgt bei der Definition eines Untersuchungsprogramms und ist im Wesentlichen abhängig von einer medizinischen Fragestellung, der betrachteten Körperregion und/oder besonderen anatomischen Gegebenheiten eines Untersuchungsobjektes.

Ein möglicher Betriebsmodus ist bspw. der LOC-Modus. Er sieht vor, die Fokustischposition einzustellen, sofern die Fokusposition für einen betrachteten Messschritt einen definierten Maximal-Abstand zur Isozentrumsposition einhält. Nur dann, wenn der Maximal-Abstand überschritten wird, wird für die Messung die Isozentrumstischischposition eingestellt, um unerwünschte Qualitätseinbußen der erzeugten Bildaufnahmen zu vermeiden. Ein anderer Betriebsmodus ist der ISO-Modus. Hierbei muss für jeden Messschritt die Isozentrumstischposition eingenommen werden.

Entsprechend würde eine erfindungsgemäße Anzeige der Übersichtsdarstellung erfolgen, wenn als Betriebsmodus der LOC-Modus eingestellt wäre. Im ISO-Modus würde eine Anzeige mit auf die Isozentrumsposition ausgerichtetem Auswahlbereich erfolgen.

Um den Betriebsmodus zu bestimmen, kann bspw. die Parameterkarte des Messprogramms bzw. der globale Positionsparameter ausgelesen werden.

Auf der Parameterkarte ist ein weiterer Positionsparameter umfasst, welcher angibt auf welche Position ein Auswahlbereich bzw. eine Schichtgruppe zur Planung ausgerichtet angezeigt werden soll. Erfindungsgemäß ist für diesen Positionsparameter die Option 'Fokusposition' vorgesehen und voreingestellt, sodass, sofern der Betriebsmodus dies erlaubt, bei der Planung eines Messschrittes der Auswahlbereich initial an der Fokusposition geöffnet wird.

Gemäß einem weiteren Ausführungsbeispiel der Erfindung kann vorgesehen sein, dass das Anzeigen der Übersichtsdarstellung des zu untersuchenden Körperbereichs mit auf eine entsprechend einer Benutzereingabe von der Fokusposition abweichenden Position ausgerichtetem Auswahlbereich erfolgt, sofern der eingestellte Betriebsmodus dies erlaubt. Mit anderen Worten besteht in dieser Ausführung die Möglichkeit für den Benutzer, die Position, zu welcher der Auswahlbereich in der Übersichtsdarstellung angezeigt wird, gleich zu Beginn ei-ner Schichtplanung und/oder währenddessen anzupassen. Auch hierfür muss als voraussetzend ein Betriebsmodus für das Messprotokoll eingestellt sein, der diese Anpassung erlaubt, bspw. der LOC-Modus. Eine von der Fokusposition abweichende Position kann dabei entsprechend einem weiteren Ausführungsbeispiel der Erfindung jede beliebige, vorab festgelegte Position innerhalb des dargestellten, zu untersuchenden Körperbereichs sein, insbesondere auch die Isozentrumsposition. Die Benutzereingabe kann dabei bspw. per Ausgabe- und Eingabeeinheit des Tomographen über ein Auswahlmenü mit mehreren vorab definierten alternativen Anzeigepositionen oder über die Eingabe von Koordinaten bezüglich eines Referenzkoordinatensystems des Tomographen erfolgen.

Gemäß einem anderen Ausführungsbeispiel der Erfindung ist auch ein Erfassen einer Benutzereingabe bezüglich einer Anpassung des für das ausgewählte Messprotokoll eingestellten Betriebsmodus, und ein Anpassen des Betriebsmodus entsprechend der Benutzereingabe umfasst. Mit anderen Worten kann erfindungsgemäß der Betriebsmodus eines Messprotokolls bzw. eines Messprogramms geändert werden, um die erfindungsgemäße Anzeige der Übersichtsdarstellung zu bewirken. Bevorzugt erfolgt die Änderung des Betriebsmodus global für alle Messprotokolle eines Messprogramms. Bspw. kann erfindungsgemäß der Benutzer einen eingestellten ISO-Modus in einen LOC-Modus ändern.

In einem bevorzugten Ausführungsbeispiel umfasst das Anzeigen der Übersichtsdarstellung des zu untersuchenden Körperbereichs einen Indikator, der die in Abhängigkeit des Prüfkriteriums einzustellende Tischposition anzeigt. Anders ausgedrückt umfasst die Übersichtsdarstellung gleichzeitig eine Anzeige, ob für die geplante Messung in Abhängigkeit der aktuell eingestellten veränderten Fokusposition die Fokustischposition oder die Isozentrumstischposition eingestellt würde. Derart erfolgt eine direkte und intuitive Veranschaulichung des Prüfergebnisses für den Benutzer. Der Indikator kann bspw. in Form eines zusätzlichen Buttons ausgebildet sein, welcher verschiedenfarbig gefüllt ist, je nachdem, welche Tischposition eingestellt wird. Alternativ kann der Indikator durch das die Fokusposition kennzeichnende Dreieck gebildet werden, welches in Abhängigkeit der einzustellenden Tischposition entweder leer oder gefüllt ist. Alternative Ausgestaltungen des Indikators sind ebenfalls möglich.

Denkbar ist in einem weiteren Aspekt eine Recheneinheit zum Einstellen einer Tischposition für einen Tisch eines Tomographen zur Untersuchung eines Körperbereichs eines auf dem Tisch befindlichen Untersuchungsobjektes, aufweisend Mittel zum Durchführen des erfindungsgemäßen Verfahrens.

Denkbar ist in einem weiteren Aspekt ein Computerprogramm mit Programmcode, um das erfindungsgemäße Verfahren zum Einstellen einer Tischposition für einen Tisch eines Tomographen zur Untersuchung eines Körperbereichs eines auf dem Tisch befindlichen Untersuchungsobjektes durchzuführen, wenn das Computerprogramm auf einem Computer ausgeführt wird.

Denkbar ist in einem weiteren Aspekt einen computerlesbaren Datenträger mit Programmcode eines Computerprogramms, um das erfindungsgemäße Verfahren zum Einstellen einer Tischposition für einen Tisch eines Tomographen zur Untersuchung eines Körperbereichs eines auf dem Tisch befindlichen Untersuchungsobjektes durchzuführen, wenn das Computerprogramm auf einem Computer ausgeführt wird.

Die Erfindung betrifft in einem weiteren Aspekt auch einen Tomographen zum Einstellen einer Tischposition für einen Tisch eines Tomographen zur Untersuchung eines Körperbereichs eines auf dem Tisch befindlichen Untersuchungsobjektes mit einer Recheneinheit. Der Tomograph ist bevorzugt ein Magnetresonanztomograph. Vorteilhaft ist die Recheneinheit in die medizinische Bildgebungsanlage integriert. Alternativ kann die Recheneinheit auch entfernt bzw. abgelegen angeordnet sein. Die Recheneinheit kann ausge-bildet sein, das erfindungsgemäße Verfahren, für einen Tomographen oder für eine Vielzahl von medizinischen Anlagen durchzuführen, z.B. in einem Radiologie-Zentrum oder Krankenhaus umfassend mehrere Magnetresonanztomographen.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden. Durch diese Beschreibung erfolgt keine Beschränkung der Erfindung auf diese Ausführungsbeispiele. In verschiedenen Figuren sind gleiche Komponenten mit identischen Bezugszeichen versehen. Die Figuren sind in der Regel nicht maßstäblich. Es zeigen:
- FIG 1: eine Ansicht eines erfindungsgemäßen Tomographen in Form eines Magnetresonanztomographen gemäß einem Ausführungsbeispiel,
- FIG 2: eine schematische Darstellung eines erfindungsgemäßen Verfahrens gemäß einem Ausführungsbeispiel,
- FIG 3: eine Übersichtsdarstellung eines zu untersuchenden Körperbereichs gemäß einem Ausführungsbeispiel der Erfindung,
- FIG 4: eine weitere Übersichtsdarstellung eines zu untersuchenden Körperbereichs gemäß einem anderen Ausführungsbeispiel der Erfindung, und
- FIG 5: eine weitere Übersichtsdarstellung eines zu untersuchenden Körperbereichs gemäß einem weiteren Ausführungsbeispiel der Erfindung.

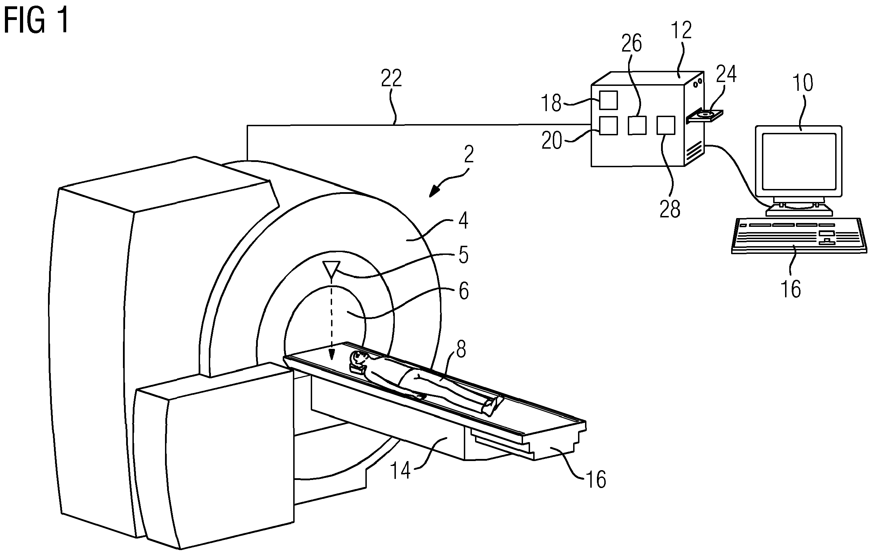

Der in Figur 1 gezeigte erfindungsgemäße Tomograph 2 in Form eines Magnetresonanztomographen umfasst eine hohlzylinderförmige Basiseinheit 4, in deren Innerem, dem sogenannten Tunnel 6, im Betrieb ein elektromagnetisches Feld für eine Magnetresonanz-Messung bzw. Untersuchung eines Untersuchungsobjektes in Form eines Patienten 8 erzeugt wird. Weiter ist ein Patiententisch 14 mit einem verfahrbaren Liegebrett 16 vorgesehen. Der Patient 8 kann auf dem Liegebrett 16 bspw. wie abgebildet positioniert werden. Der Patiententisch 14 ist außerhalb der Basis-einheit 4 so positioniert, dass das Liegebrett 16 samt Patient 8 zumindest teilweise für die Untersuchung in den Tunnel 6 hineingefahren werden kann. Am Gehäuse der Basiseinheit 4 oberhalb des Eingangs des Tunnels 6 angeordnet ist ein Lasermarker 5 bzw. ein Markierungslaser in Form einer Laserquelle angeordnet, dessen Laserstrahl (gestrichelter Pfeil) sich vertikal nach unten auf die Patientenliege 16 ausbreitet. Der Lasermarker 6 kann zur erfindungsgemäßen Festlegung der Isozentrumsposition dienen.

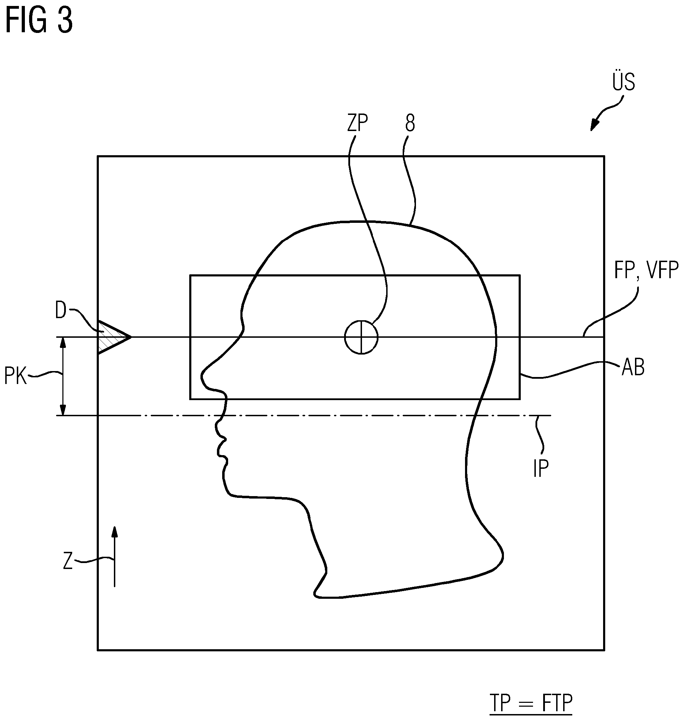

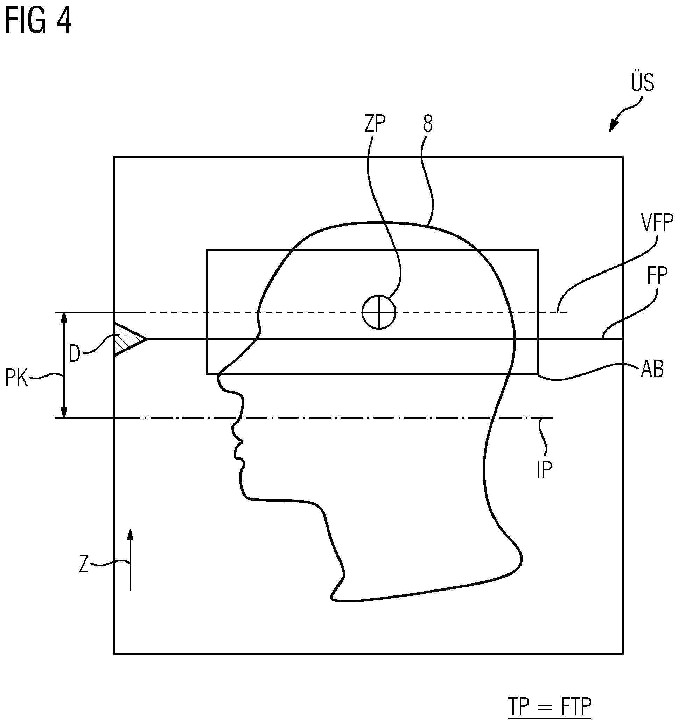

Der Tomograph 2 verfügt über eine Recheneinheit in Form eines Computersystems 12, welches als Computer und zur Ausführung des erfindungsgemäßen Verfahrens ausgebildet ist. Die Recheneinheit 12 ist entsprechend mit einer Anzeigeeinheit 10, beispielsweise zur graphischen Anzeige einer erfindungsgemäßen Übersichtdarstellung, wie bspw. in Figur 3 oder Figur 4 sowie einer Eingabeeinheit 16 verbunden. Bei der Anzeigeeinheit 10 kann es sich beispielsweise um einen LCD-, Plasma- oder OLED-Bildschirm handeln. Es kann sich weiterhin um einen berührungsempfindlichen Bildschirm handeln, welcher auch als Eingabeeinheit 16 ausgebildet ist. Ein solcher berührungsempfindlicher Bildschirm kann in das bildgebende Gerät integriert oder als Teil eines mobilen und/oder portablen Geräts ausgebildet sein. Bei der Eingabeeinheit 10 handelt es sich beispielsweise um eine Tastatur, eine Maus, einen sogenannten "Touch-Screen" oder auch um ein Mikrofon zur Spracheingabe. Die Eingabeeinheit 16 kann auch eingerichtet sein, um Bewegungen eines Benutzers zu erkennen und in entsprechende Befehle zu übersetzen. Über die Anzeigeeinheit 10 und/oder die Eingabeeinheit 16 können erfindungsgemäß verschiedene, durch den Benutzer vorgebbare Positionen, insbesondere die Isozentrumsposition, die Fokusposition und/oder eine veränderte Fokusposition erfasst und an die Recheneinheit 12 auf bekannte Weise kabellos oder kabelgebunden übertragen werden. Zusätzlich können auch Benutzer-seitige Angaben über die Änderung eines Betriebsmodus über die Anzeigeeinheit 10 und/oder die Eingabeeinheit 16 erfasst und der Recheneinheit 12 zur Verfügung gestellt werden.

Das Computersystem steht auch mit der Basiseinheit 4 des Tomographen 2 zum Datenaustausch in Verbindung. Es können bspw. Steuersignale für den Tomographen 2 für die Magnetresonanz-Untersuchung vom Computersystem 12 an die Basiseinheit 4 übertragen werden. Dazu können bspw. verschiedene, jeweils auf eine Untersuchungsart abgestimmte Messprotokolle in einem Speicher 18 hinterlegt sein und durch einen Nutzer, bspw. einen Arzt oder medizinisch-technisch geschultes Personal, vor der Messung entsprechend einer medizinischen Fragestellung ausgewählt werden. Dazu können verschiedene Messprotokolle, insbesondere verschiedene Messprotokolle eines Messprogramms, dem Nutzer über die Anzeigeeinheit 10 zur Auswahl ausgegeben werden und der Nutzer kann eines der Messprotokolle über die Eingabeeinheit 16 auswählen, bspw. per Maus-Click. Alternativ kann ein Messprotokoll auch über ein Intranet oder Internet von einer zentralen Vorgabeeinheit (nicht dargestellt) über entsprechende Datenverbindungen automatisch oder remote durch einen entfernten Nutzer an dem Tomographen 2 zur Verfügung gestellt werden. Anhand des gewählten Messprotokolls wird der Tomograph 2 für die Untersuchung eingestellt. Insbesondere wird die erfindungsgemäß ermittelte Tischposition am Tomographen 2 eingestellt. Die Ansteuerung der Basiseinheit 4 erfolgt entsprechend des gewählten Messprotokolls. Andererseits werden aufgenommene Messdaten für eine Weiterverarbeitung in einer nicht näher beschriebenen Rekonstruktionseinheit 20 erfasst. Die Verbindung 22 ist in bekannter Weise kabelgebunden oder kabellos über entsprechende Schnittstellen realisiert.

Das Computersystem 12 umfasst auch eine Ermittlungseinheit 26. Diese ist eingerichtet, um die Benutzer-seitigen Eingaben bezüglich einer Isozentrumsposition und/oder einer Fokusposition, ggf. unter Berücksichtigung einer Relativlage des Lasermarkers 5 und eines Isozentrums des Tomographen 2 in Bezug zu einem Referenzkoordinatensystem des Tomographen für einen auf der Patientenliege 16 befindlichen Patienten 8 in jeweils eine Isozentrumstischposition und eine Fokustischposition zu überführen.

Das Computersystem 12 umfasst ferner eine Prüfeinheit 28, die einerseits dazu dient, einen Betriebsmodus für ein ausgewähltes Messprotokoll zu bestimmen und in Abhängigkeit von Ergebnis festzulegen, auf welche Position ein Auswahlbereich in einer Übersichtsdarstellung ausgerichtet anzuzeigen ist. Andererseits dient die Prüfeinheit 28 dazu, zu prüfen, ob ein Höchstmaß für ein Prüfkriterium hinsichtlich einer veränderten Fokusposition eingehalten oder verletzt wird. Insbesondere kann es sich bei dem Prüfkriterium um ein Abstandsmaß zwischen Isozentrumsposition und veränderter Fokusposition handeln. Anders ausgedrückt wird ein Abstand zwischen dem Isozentrum und einem der veränderten Fokusposition entsprechender Körperposition des Patienten betrachtet, wenn eine Tischposition entsprechend der veränderten Fokusposition eingestellt würde. Alternativ wird ein Abstand zwischen der Isozentrumsposition und einer maximal von der Isozentrumsposition entfernten Position innerhalb des Auswahlbereichs betrachtet. Das Höchstmaß kann bspw. im Speicher 18 des Tomographen 2 abrufbar für die Prüfeinheit 28 hinterlegt sein. Das Höchstmaß ist insbesondere abhängig von dem ausgewählten Messprotokoll, also der zugrunde liegenden, medizinischen Fragestellung, der damit betrachteten anatomischen Region und oder den technischen Gegebenheiten des Tomographen 2, insbesondere der Ausdehnung des homogenen Teils seines Magnetfeldes. Die Prüfeinheit 28 ist ferner eingerichtet, Steuersignale in Abhängigkeit des Prüfergebnisses für den Tomographen 2 zu erzeugen und an diesen zu übermitteln, mittels welcher die Tischposition für die geplante Messung eingestellt wird. Bspw. kann die Prüfeinheit 28 als Steuersignal ausgeben, dass die Fokustischposition eingestellt werden soll, falls das Prüfkriterium erfüllt, also das Abstands-Höchstmaß eingehalten wurde. Wird das Abstands-Höchstmaß überschritten bzw. verletzt, gibt die Prüfeinheit 28 als Steuersignal aus, dass die Isozentrumstischposition eingestellt werden soll.

Vorliegend sind insbesondere die genannten Einheiten 26 und 28 als getrennte Module innerhalb des Computersystems 12 ausgestaltet, die, wo erforderlich, in Datenaustausch miteinander stehen. Alternativ können alle genannten Einheiten als eine Recheneinheit integriert sein, sei es in Form einer körperlichen oder funktionalen Integrität.

Das Computersystem 12 kann mit einem computerlesbaren Datenträger 24 zusammenwirken, insbesondere um durch ein Computerprogramm mit Programmcode ein erfindungsgemäßes Verfahren durchzuführen. Weiterhin kann das Computerprogramm auf dem maschinenlesbaren Träger 24 abrufbar gespeichert sein. Insbesondere kann es sich bei dem maschinenlesbaren Träger 24 um eine CD, DVD, Blu-Ray Disc, einen Memory-Stick oder eine Festplatte handeln.

Insbesondere die Einheiten 26 und 28 können in Form von Hard- oder in Form von Software ausgebildet sein. Beispielsweise sind die Einheiten als sogenannte FPGAs (Akronym für das englischsprachige "Field Programmable Gate Array") ausgebildet oder umfassen eine arithmetische Logikeinheit.

Auf dem Speicher 18 des Computersystems 12 kann wenigstens ein Computerprogramm gespeichert sein, welches alle Verfahrensschritte des erfindungsgemäßen Verfahrens durchführt, wenn das Computerprogramm auf dem Computer ausgeführt wird. Das Computerprogramm zur Ausführung der Verfahrensschritte des erfindungsgemäßen Verfahrens umfasst Programmcode. Weiterhin kann das Computerprogramm als ausführbare Datei ausgebildet sein und/oder auf einem anderen Rechensystem als dem Computersystem 12 gespeichert sein. Beispielsweise kann der Magnetresonanztomograph 2 so ausgelegt sein, dass das Computersystem 12 das Computerprogramm zum Ausführen des erfindungsgemäßen Verfahrens über ein Intranet oder über das Internet in seinen internen Arbeitsspeicher lädt. Alternativ kann vorgesehen sein, dass das Computersystem 12 selbst Teil eines Internets oder Intranets, bspw. eines HIS (Hospital Information System) oder eines RIS (Radiology Information System) ist und Zugriff auf eingegebene, ausgewählte oder zentral hinterlegte Messprotokolle verschiedener MagnetresonanzTomographen 2 der Einrichtung hat, um das erfindungsgemäße Verfahren zentral für verschiedene Tomographen 2 auszuführen.

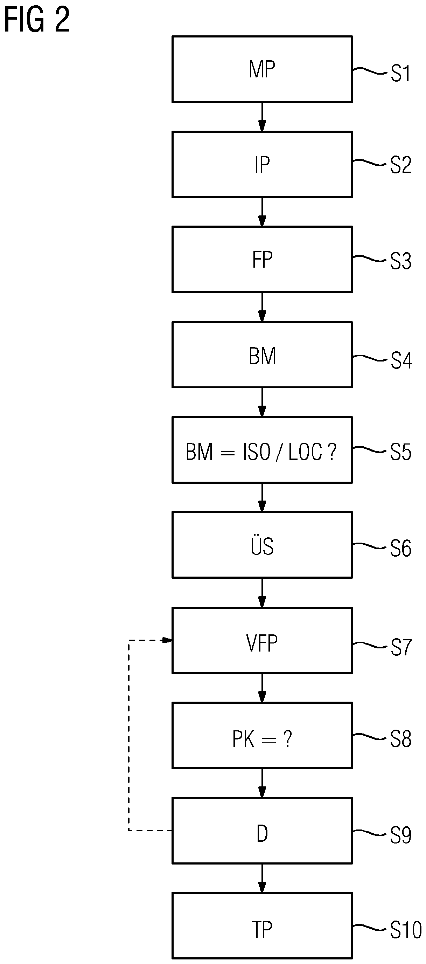

Das in Figur 2 gezeigte erfindungsgemäße Verfahren umfasst eine Vielzahl von Schritten. Es dient der vereinfachten und beschleunigten Einstellung einer Tischposition TP eines Tomographen 2 für eine Untersuchung. Kern des erfindungsgemäßen Verfahrens ist es, bei der Protokollplanung angezeigten Auswahlbereich AB oder Field of View des Tomographen 2 der in aller Regel auch die mit einem Messprotokoll MP zu erfassende Schichtgruppe umfasst, auf eine vorab definierte Fokusposition FP auszurichten. Bei der Planung braucht der Benutzer derart keine oder nur noch geringfügige Anpassungen der einzustellenden Tischposition vorzunehmen. Dies beschleunigt die Messplanung und erhöht den Komfort für den Patienten 8, der sich während der Planung eines jeden Messschrittes auf der Patientenliege 16 des Tisches 14 befindet.

Die Schritte S1, S2 und S3 des Verfahrens sind an sich bekannt und werden daher nur kurz erläutert. In Schritt S1 erfolgt eine Auswahl eines Messprotokolls MP aus einer Vielzahl von Messprotokollen. In der Regel entspricht dabei die Vielzahl von Messprotokollen MP allen von einem Messprogramm zur Untersuchung des Patienten 8 zur Beantwortung einer konkreten medizinischen Fragestellung umfassten Messprotokolle. Die Auswahl kann durch einen Benutzer erfolgen, bspw. durch Auswahl aus einer Liste oder durch automatischen Vorschlag und Nutzer-Bestätigung oder dergleichen. In Schritt S2 wird eine Isozentrumsposition IP für das ausgewählte Messprotokoll MP definiert bzw. festgelegt. Dies kann bspw. mittels oben beschriebenem Lasermarker 5 des Tomographen 2 erfolgen. Der Benutzer markiert dabei also den Teil, Bereich, Abschnitt oder ein Organ, welches bei der Messung im Isozentrum des Tomographen 2 angeordnet sein soll. In Schritt S2 kann auch eine Aufnahme einer Übersichtsaufnahme ÜS mit dem Tomographen 2 erfolgen. Die Übersichtsaufnahme ÜS bildet den zu untersuchenden Körperbereich ab, indem die Tischposition TP auf eine Isozentrumstischposition ITP entsprechend der Isozentrumsposition IP eingestellt wird, die der Benutzer zuvor markiert hat. Man spricht hierbei auch von der O-Position. Bei der Isozentrumstischposition ITP spricht man auch von der O-Lage des Patienten 8. Die Übersichtsaufnahme ÜS kann so der Überprüfung der Isozentrumsposition IP dienen. In Schritt S3 wird eine von der Isozentrumsposition IP abweichende Fokusposition FP durch den Benutzer festgelegt. Die Fokusposition FP ist per Definition die Position relativ zur Anatomie des Patienten 8 in einer zu untersuchenden Körperregion, an der eine Untersuchung durchgeführt werden soll. Dazu kann dem Benutzer bspw. über eine Interaktionsschnittstelle, bspw. gebildet durch die Anzeigeeinheit 10 und die Eingabeeinheit 16, bspw. ein Graphical User Interface (im folgenden GSP genannt), die Übersichtsaufnahme ÜS samt Isozentrumsposition IP angezeigt werden. In der Übersichtsaufnahme ÜS kann er nun eine Fokusposition FP festlegen, indem er bspw. per Mausklick eine gewünschte Position in der Übersichtsaufnahme ÜS markiert.

In einem Schritt S4 wird ein Betriebsmodus BM ermittelt, welcher für das ausgewählte Messprotokoll MP eingestellt ist. Dazu kann bspw. ein globaler Positionierungsparameter des Messprogramms MP ausgelesen werden. Im Folgenden wird im Sinne der Erfindung davon ausgegangen, dass für das ausgewählte Messprotokoll MP ein Betriebsmodus BM eingestellt ist, der Messungen zulässt, bei denen der Patiententisch 14, 16 nicht auf die Isozentrumstischposition ITP eingestellt werden muss, bspw. dann, wenn das Messprogramm eine Körperregion des Patienten 8 betrifft, die auch bei von der Isozentrumstischposition ITP im Rahmen einer vorab definierten Toleranz abweichender Tischposition TP eine ausreichend gute Bildqualität liefert. Ein solcher Betriebsmodus BM ist bspw. der LOC-Modus. Im Gegensatz dazu ist der ISO-Modus ein Betriebsmodus, in dem bei jedem Messschritt die Isozentrumstischposition ITP eingestellt werden muss.

Erfindungsgemäß kann ein optionaler Schritt S5 vorgesehen sein, in welchem der Benutzer den Betriebsmodus BM des ausgewählten Messprotokolls MP anpassen bzw. verändern kann. In diesem ISO-/LOC-Interaktionsschritt hat der Benutzer also die Möglichkeit, über die Anpassung des globalen Positionierungsparameters des Messprogramms im Rahmen der Protokollplanung zwischen verschiedenen Betriebsmodi zu wechseln. Insbesondere kann er von einem voreingestellten ISO-Modus in einen LOC-Modus wechseln, um eine erfindungsgemäße Anzeige der Übersichtsdarstellung im GSP gemäß Schritt S6 zu erzielen. Eine derartige Änderung des Positionierungsparameters ändert den Betriebsmodus BM für alle folgenden Messschritte. Schritt S5 kann bspw. über ein Auswahlmenü des GSP erfolgen.

In einem Schritt S6 erfolgt erfindungsgemäß eine Anzeige einer Übersichtsdarstellung ÜS darstellend den zu untersuchenden Körperbereich im GSP umfassend den mit dem geplanten Messprotokoll MP abzubildenden Auswahlbereich AB und eine Anzeige der Fokusposition FP. Eine entsprechende Anzeige ist in Figur 3 veranschaulicht. Bei dem zu untersuchenden Körperbereich handelt es sich hier um den Kopf bzw. Hals des Patienten 8. Die Fokusposition FP wird in der Anzeige als quer zur Längsachse des Patienten 8, also quer zur Verstellrichtung (Z-Achse) des Patiententisches 14, 16 verlaufende Linie veranschaulicht. Zudem wird die Fokusposition FP mittels eines am Rand der Übersichtsdarstellung angeordneten Indikators D in Form eines Dreiecks markiert, dessen eine Ecke auf die Z-Position der Fokuspositions-Linie zeigt. Erfindungsgemäß wird bei der Anzeige der Auswahlbereich AB von vorn herein auf die Fokusposition FP ausgerichtet. Mit anderen Worten ist der Auswahlbereich AB derart angeordnet, dass bspw. ein vom Auswahlbereich AB umfasstes Schichtgruppenzentrum, welches durch eine Zentrumsposition ZP des Auswahlbereichs verläuft, über der Fokusposition FP liegt. Daneben umfasst vorliegend die Anzeige der Übersichtsdarstellung ÜS auch eine Anzeige der Isozentrumsposition IP, dargestellt durch eine punktgestrichelte Linie.

Mit anderen Worten wird auf der Routine Parameter Karte des ausgewählten Messprotokolls MP erfindungsgemäß ein Positionsparameter bereitgestellt, der angibt, an welcher Position ein Auswahlbereich AB bzw. ein Schichtgruppenzentrum ausgerichtet bei der Protokollplanung angezeigt werden soll. Dieser Positionsparameter wird erfindungsgemäß default-mäßig auf, Fokusposition' gesetzt. Im Einzelfall kann vorgesehen sein, dass der Benutzer über ein Auswahlmenü den Positionsparameter anpasst und bspw. die Isozentrumsposition IP oder eine beliebige andere Position als die Anzeigeposition wählt.

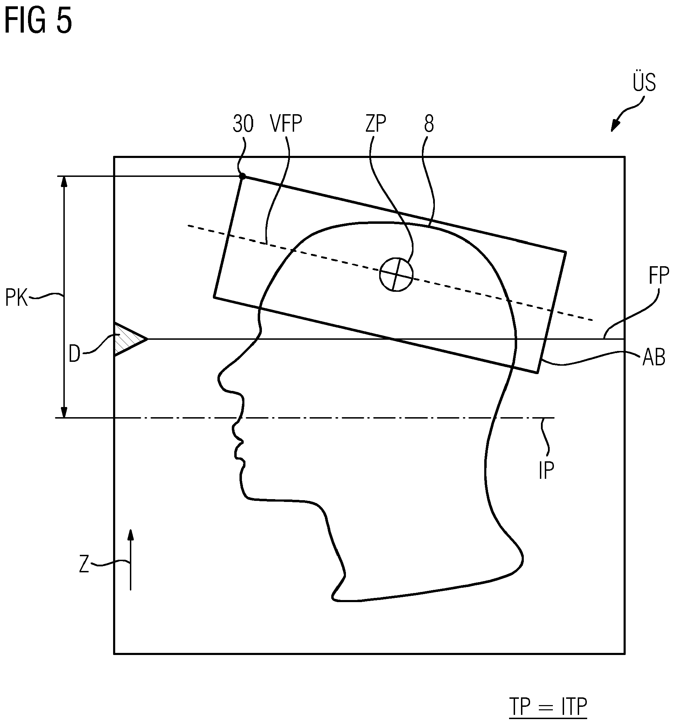

In einem Folgeschritt S7 kann der Benutzer über das GSP direkt in der Übersichtsdarstellung ÜS eine veränderte Fokusposition VFP auswählen. Dazu kann er bspw. den Auswahlbereich AB entlang der Z-Achse verschieben, bis sein Zentrum ZP auf einer gewünschten Position, die veränderte Fokusposition VFP, zu liegen kommt. Eine Verschiebung kann zusätzlich oder alternativ auch in eine andere Raumrichtung X oder Y erfolgen. Die veränderte Fokusposition VFP kann sich zudem durch eine Drehung bzw. Kippung des Auswahlbereichs AB um einen Zentrumspunkt ZP ergeben. Alternativ kann der Benutzer auch Koordinaten oder ein Gradzahl für eine Drehung eingeben, wonach dann bspw. um das Zentrum ZP der Auswahlbereich AB verschoben bzw. gedreht wird. In aller Regel liegt die veränderte Fokusposition VFP sehr dicht, jedenfalls deutlich dichter als die Isozentrumsposition IP, bei der Fokusposition FP. Mit anderen Worten sind die erforderlichen Manipulationen durch den Benutzer nur geringfügig. In einer weiteren Alternative für Schritt S7, wie sie in der Praxis häufig auftritt, kann der Benutzer die bestehende Fokusposition FP bestätigen. Mit anderen Worten entspricht in dieser Alternative die veränderte Fokusposition VFP der Fokusposition FP. Dies ist ebenfalls in Figur 3 veranschaulicht. Eine Verschiebung des Auswahlbereichs AB erübrigt sich, was die Protokollplanung vereinfacht und beschleunigt. Hingegen zeigen die Figur 4 sowie die Figur 5 eine Anzeige einer Übersichtsdarstellung ÜS, in welcher der Auswahlbereich AB bzw. die veränderte Fokusposition VFP entsprechend einer Benutzereingabe gegenüber der Fokusposition FP verschoben wurde.

In einem Schritt S8 erfolgt eine Überprüfung eines Prüfkriteriums PK. Das Prüfkriterium PK umfasst bzw. berücksichtigt, wie in Figur 3 und Figur 4 durch einen zweiseitigen Pfeil verdeutlicht, ein Abstandsmaß zwischen Isozentrumsposition IP und veränderter Fokusposition VFP. Dazu wird der Abstand zwischen Isozentrumsposition IP und veränderter Fokusposition VFP ermittelt und mit einem Höchstmaß für den Abstand verglichen und ein Ergebnis abgeleitet. Das Prüfkriterium PK kann alternativ oder zusätzlich, wie in Figur 5 ebenfalls durch einen zweiseitigen Pfeil veranschaulicht, auch ein Abstandsmaß zwischen Isozentrumsposition IP und einer maximal von derselben entfernten Position 30 innerhalb des Auswahlbereichs AB berücksichtigen. Dazu wird der Abstand zwischen Isozentrumsposition IP und der maximal entfernten Position 30 ermittelt und mit einem Höchstmaß für diesen Abstand verglichen und ein Ergebnis abgeleitet. Das Höchstmaß kann jeweils zum einen vom ausgewählten Messprotokoll, der medizinischen Fragestellung, der betrachteten Körperregion, oder dergleichen abhängen. Zum anderen kann sich das Höchstmaß auch aus technischen Merkmalen des Tomographen 2 selbst ableiten, bspw. der Ausdehnung des im Wesentlichen homogenen Magnetfeldbereiches innerhalb des Tunnels 6. Insofern kann das Höchstmaß auch Information über die Abbildungsqualität des Tomographen 2 umfassen. Ein jeweiliger Wert für das Höchstmaß kann bspw. im Speicher 18 der Recheneinheit 12 oder auch auf einem zentralen Speicher, bspw. eines Krankenhauses abrufbar hinterlegt sein.

In einem Schritt S9 wird dem Benutzer das Prüfergebnis bezüglich der veränderten Fokusposition VFP über das GSP angezeigt. Ergibt die Prüfung, dass der Abstand zwischen Isozentrumsposition IP und veränderter Fokusposition VFP bzw. der maximal beabstandeten Position 30 des Auswahlbereichs AB kleiner oder gleich dem Höchstmaß ist, so folgt als für die Messung einzustellende Tischposition TP die Fokustischposition FTP. Mit anderen Worten liegt der Abstand von Isozentrumsposition IP und veränderter Fokusposition VFP innerhalb der Toleranz mit akzeptabler Bildqualität. In diesem Fall wird, wie in Figur 3 und Figur 4 zutreffend, das die in der Übersichtsdarstellung ÜS Fokusposition FP markierende Dreieck D bspw. farblich gefüllt dargestellt (Schraffur). Ergibt die Prüfung hingeben, größer dem Höchstmaß ist, so folgt als für die Messung einzustellende Tischposition TP die Isozentrumstischposition ITP. Mit anderen Worten liegt der Abstand außerhalb der Toleranz, eine Bildaufnahme würde hier eine inakzeptable Bildqualität liefern. In diesem Fall wird das die in der Übersichtsdarstellung ÜS Fokusposition FP markierende Dreieck D bspw. leer, sprich nur als Rand-linie, dargestellt, wie in Figur 5 dargestellt. Der Benutzer kann so direkt für die zuvor ausgewählte, veränderte Fokusposition VFP erkennen, welche Tischposition TP eingestellt würde. Entspricht das derart dargestellte Prüfergebnis nicht den Wünschen des Benutzers, hat er die Möglichkeit, über eine Wiederholschleife (gestrichelter Pfeil in Fig. 2) die Schritte S7, s8 und S9 zu wiederholen, und zwar so oft, bis er das gewünschte Prüfergebnis erhält.

In einem Schritt S10 wird ein dem Prüfergebnis entsprechendes Steuersignal für den Tomographen 2 erzeugt und an diesen für eine Einstellung der Tischposition TP entweder auf die Isozentrumstischposition ITP oder die Fokustischposition FTP übertragen und die Tischposition TP entsprechend eingestellt.

Mit dieser Tischposition TP wird im Folgenden die Messung für den betrachteten Messschritt durchgeführt. Das beschriebene Verfahren kann danach für jeden weiteren Messschritt wiederholt werden.

Ein möglicher Workflow könnte also zusammenfassend folgendermaßen aussehen: Definition einer Isozentrumsposition IP mittels Laser, bspw. auf dem Herzen eines Patienten 8. Da die Lasermarkierung jedoch nur eine eingeschränkte Genauigkeit erlaubt, wird nach der ersten Messung eines Localizer-Bildes bzw. einer Übersichtsdarstellung ÜS eine Fokusposition FP in einem GSP mittels Fokus-Dreieck D definiert bzw. präzisiert. Ein Positionsparameter auf der Parameterkarte wird erfindungsgemäß auf die Fokusposition FP eingestellt. Alle danach zu messenden Protokolle MP werden dann im GSP bei der Planung direkt auf der Fokusposition FP geöffnet, und nicht mehr wie bisher, auf der Isozentrumsposition IP. Dies ist insofern vorteilhaft, als Workflows, die verschiedene Tischpositionen TP nutzen, derart aufgesetzt werden können, dass Schichtgruppen nicht mehr für jeden Messschritt einzeln in Richtung der Fokusposition FP verschoben werden müssen.

## Patentansprüche

1. Verfahren zum Einstellen einer Tischposition (TP) für einen Tisch (14, 16) eines Tomographen (2) zur Messung eines Körperbereichs eines auf dem Tisch befindlichen Untersuchungsobjektes (8), mit den Schritten:
- Erfassen (S1) einer Benutzerauswahl eines Messprotokolls (MP) aus einer Mehrzahl möglicher Messprotokolle,
- Erfassen (S2) einer Benutzereingabe bezüglich einer mittels Lasermarker festgelegten Isozentrumsposition (IP),
- Erfassen (S3) einer Benutzereingabe, nämlich Markieren in einem Localizer-Bild, bezüglich einer Fokusposition (FP),
- Anzeigen (S6) einer Übersichtsdarstellung (ÜS) des zu untersuchenden Körperbereichs umfassend einen Auswahlbereich (AB) und einen Indikator (D) der Fokusposition relativ zu dem Körperbereich, wobei der Auswahlbereich auf die Fokusposition ausgerichtet angezeigt wird,
- Erfassen (S7) einer Benutzereingabe bezüglich einer veränderten Fokusposition (VFP),
- Ausrichten (S8) des Auswahlbereichs auf die veränderte Fokusposition, und
- Einstellen (S10) der Tischposition auf eine Fokustischposition (FTP) entsprechend der Fokusposition, sofern die veränderte Fokusposition ein Prüfkriterium (PK) erfüllt, und Einstellen (S10) auf eine Isozentrumstischposition (ITP) entsprechend der Isozentrumsposition, sofern die veränderte Fokusposition das Prüfkriterium (PK) verletzt.

2. Verfahren nach Anspruch 1, wobei das Prüfkriterium ein Abstandsmaß zwischen der veränderten Fokusposition und der Isozentrumsposition berücksichtigt und erfüllt ist, sofern das Abstandsmaß ein vorab festgelegtes Höchstmaß nicht überschreitet.

3. Verfahren nach Anspruch 1 oder 2, wobei das Prüfkriterium ein Abstandsmaß zwischen einem von der Isozentrumsposition am weitesten entfernten Punkt des Auswahlbereichs und der Isozentrumsposition berücksichtigt und erfüllt ist, sofern das Abstandmaß ein vorab festgelegtes Höchstmaß nicht überschreitet.

4. Verfahren nach Anspruch 2 oder 3, wobei das vorab festgelegte Höchstmaß abhängig von einer anatomischen Information und/oder einer Information bezüglich der Homogenität eines Magnetfeldes des Tomographen ist.

5. Verfahren nach einem der vorhergehenden Ansprüche, wobei das Anzeigen der Übersichtsdarstellung des zu untersuchenden Körperbereichs mit auf die Fokusposition ausgerichtetem Auswahlbereich erfolgt, sofern ein für das ausgewählte Messprotokoll eingestellter Betriebsmodus (BM) dies vorsieht.

6. Verfahren nach Anspruch 5, wobei das Anzeigen der Übersichtsdarstellung des zu untersuchenden Körperbereichs mit auf eine entsprechend einer Benutzereingabe von der Fokusposition abweichenden Position ausgerichtetem Auswahlbereich erfolgt, sofern der eingestellte Betriebsmodus dies erlaubt.

7. Verfahren nach Anspruch 6, wobei die von der Fokusposition abweichende Position die Isozentrumsposition oder eine beliebige vorab festgelegte Position innerhalb des zu untersuchenden Körperbereichs ist.

8. Verfahren nach einem der vorhergehenden Ansprüche, umfassend ein
- Erfassen (S5) einer Benutzereingabe bezüglich einer Anpassung des für das ausgewählte Messprotokoll eingestellten Betriebsmodus, und
- Anpassen (S5) des Betriebsmodus entsprechend der Benutzereingabe.

9. Verfahren nach einem der vorherigen Ansprüche, wobei das Anzeigen der Übersichtsdarstellung des zu untersuchenden Körperbereichs einen Indikator (D) umfasst, der die in Abhängigkeit des Prüfkriteriums einzustellende Tischposition anzeigt.

10. Tomograph (2) zum Einstellen einer Tischposition (TP) für einen Tisch (14, 16) desselben zur Untersuchung eines Körperbereichs eines auf dem Tisch befindlichen Untersuchungsobjektes (8) mit einer als ein Computersystem ausgebildeten Recheneinheit (12) zum Einstellen einer Tischposition (TP) für den Tisch (14, 15) des Tomographen (2) zur Untersuchung des Körperbereichs des auf dem Tisch befindlichen Untersuchungsobjektes (8), wobei die Recheneinheit (12) mit einer Anzeigeeinheit (10) zur Anzeige einer Übersichtsdarstellung (ÜS) und einer Eingabeeinheit (16) verbunden ist und Mittel zum Durchführen eines Verfahrens gemäß einem der Ansprüche 1 bis 9 aufweist, wobei zum Einstellen der Tischposition eine entsprechende Ansteuerung des Tisches (14, 16) erfolgt.

## Claims

1. Method for adjusting a couch position (CP) for a couch (14, 16) of a tomograph (2) for scanning a body region of an examination object (8) situated on the couch, having the following steps:
- acquisition (S1) of a user selection of a scanning protocol (SP) from a plurality of possible scanning protocols,
- acquisition (S2) of a user input in respect of an isocenter position (IP) defined by means of laser markers,
- acquisition (S3) of a user input, namely marking in a localiser image, in respect of a focus position (FP),
- displaying (S6) an overview (OV) of the body region to be examined, comprising a selection range (SR) and an indicator (T) of the focus position relative to the body region, wherein the selection range is displayed aligned with the focus position,
- acquisition (S7) of a user input in respect of a changed focus position (CFP),
- aligning (S8) the selection range with the changed focus position, and
- adjusting (S10) the couch position to a focus couch position (FCP) corresponding to the focus position if the changed focus position meets a test criterion (TC), and adjusting (S10) to an isocenter couch position (ICP) corresponding to the isocenter position if the changed focus position does not meet the test criterion (TC).

2. Method according to claim 1, wherein the test criterion takes into account a distance measurement between the changed focus position and the isocenter position and is met if the distance measurement does not exceed a previously defined upper limit.

3. Method according to claim 1 or 2, wherein the test criterion takes into account a distance measurement between a point of the selection range furthest away from the isocenter position and the isocenter position and is met if the distance measurement does not exceed a previously defined upper limit.

4. Method according to claim 2 or 3, wherein the previously defined upper limit is dependent on an item of anatomical information and/or an item of information in respect of the homogeneity of a magnetic field of the tomograph.

5. Method according to one of the preceding claims, wherein the overview of the body region to be examined is displayed with a selection range aligned with the focus position if an operating mode (OM) adjusted for the selected scanning protocol provides for this.

6. Method according to claim 5, wherein the overview of the body region to be examined is displayed with a selection range aligned with a position that differs from the focus position corresponding to a user input if the adjusted operating mode allows this.

7. Method according to claim 6, wherein the position that differs from the focus position is the isocenter position or any previously defined position within the body region to be examined.

8. Method according to one of the preceding claims, comprising
- acquisition (S5) of a user input in respect of an adaptation of the operating mode adjusted for the selected scanning protocol, and
- adaptation (S5) of the operating mode corresponding to the user input.

9. Method according to one of the preceding claims, wherein displaying the overview of the body region to be examined comprises an indicator (T) which displays the couch position that is to be adjusted as a function of the test criterion.

10. Tomograph (2) for adjusting a couch position (CP) for a couch (14, 16) thereof for examining a body region of an examination object (8) situated on the couch, having an arithmetic unit (12) designed as a computer system for adjusting a couch position (CP) for the couch (14, 15) of the tomograph (2) for examining the body region of the examination object (8) situated on the couch, wherein the arithmetic unit (12) is connected to a display unit (10) for display of an overview (OV) and to an input unit (16) and has means for carrying out a method according to one of claims 1 to 9, wherein in order to adjust the couch position the couch (14, 16) is controlled accordingly.

## Revendications

1. Procédé de réglage de la position (TP) d'une table (14, 16) d'un tomodensitomètre (2) pour la mesure d'une partie du corps d'un objet (8) à examiner se trouvant sur la table, comprenant les stades :
- détection (S1) d'un choix d'utilisateur d'un protocole (MP) de mesure dans une pluralité de protocoles de mesure possibles,
- détection (S2) d'une entrée d'utilisateur se rapportant à une position (IP) d'isocentre fixée au moyen d'un marqueur laser,
- détection (S3) d'une entrée d'utilisateur, à savoir marquage dans une image de localisateur se rapportant à une position (FP) de foyer,
- affichage (S6) d'une représentation (ÜS) d'ensemble de la partie de corps à examiner comprenant une partie (AB) sélectionnée et un indicateur (D) de la position du foyer par rapport à la partie du corps, la partie sélectionnée étant affichée en étant orientée sur la position du foyer,
- détection (S7) d'une entrée d'utilisateur se rapportant à une position (VFP) modifiée du foyer,
- orientation (S8) de la région de sélection sur la position modifiée du foyer, et
- réglage (S10) de la position de la table à une position (FTP) de table de foyer correspondant à la position du foyer, dans la mesure où la position modifiée du foyer satisfait un critère (PK) de contrôle et réglage (S10) sur une position (ITP) de la table d'isocentre correspondant à la position d'isocentre, dans la mesure où la position modifiée du foyer contrevient au critère (PK) de contrôle.

2. Procédé suivant la revendication 1, dans lequel le critère de contrôle prend en compte une mesure de la distance entre la position modifiée du foyer et la position d'isocentre et est satisfait pourvu que la mesure de distance ne dépasse pas une mesure la plus grande fixée auparavant.

3. Procédé suivant la revendication 1 ou 2, dans lequel le critère de contrôle prend en compte une mesure de distance entre un point le plus éloigné de la position de l'isocentre de la partie sélectionnée et la position de l'isocentre et est satisfait pourvu que la distance ne dépasse pas une mesure la plus grande fixée auparavant.

4. Procédé suivant la revendication 2 ou 3, dans lequel la distance la plus grande fixée auparavant dépend d'une information anatomique et/ou d'une information concernant l'homogénéité d'un champ magnétique du tomodensitomètre.

5. Procédé suivant l'une des revendications précédentes, dans lequel l'affichage de la représentation d'ensemble de la partie du corps à examiner s'effectue par la partie sélectionnée orientée sur la position du foyer, dans la mesure où un mode (BM) de fonctionnement, réglé pour ce protocole de mesure sélectionné, le prévoit.

6. Procédé suivant la revendication 5, dans lequel l'affichage de la représentation d'ensemble de la partie du corps à examiner s'effectue par une partie sélectionnée orientée sur une position s'écartant de la position du foyer conformément à une entrée d'utilisateur, pourvu que le mode de fonctionnement réglé le permette.

7. Procédé suivant la revendication 6, dans lequel la position, s'écartant de la position du foyer, est la position de l'isocentre ou n'importe quelle position fixée auparavant au sein de la partie du corps à examiner.

8. Procédé suivant l'une des revendications précédentes, comprenant
- une détection (S5) d'une entrée d'utilisateur se rapportant à une adaptation du mode de fonctionnement réglé pour le protocole de mesure sélectionné, et
- une adaptation (S5) du mode de fonctionnement conformément à l'entrée d'utilisateur.

9. Procédé suivant l'une des revendications précédentes, dans lequel l'affichage de la représentation d'ensemble de la partie du corps à examiner comprend un indicateur (D), qui indique la position de la table se réglant en fonction du critère de contrôle.

10. Tomodensitomètre (2) pour le réglage d'une position (TP) d'une table (14, 16) de celui-ci pour l'examen d'une partie du corps d'un objet (8) à examiner se trouvant sur la table, comprenant une unité (12) informatique, constituée sous la forme d'un système d'ordinateur, pour le réglage d'une position (TP) de la table (14, 15) du tomodensitomètre (2), afin d'examiner la partie du corps de l'objet (8) à examiner se trouvant sur la table, l'unité (12) informatique étant reliée à une unité (10) d'affichage pour l'affichage d'une représentation (ÜS) d'ensemble et avec une unité (16) d'entrée et ayant des moyens pour effectuer un procédé suivant l'une des revendications 1 à 9, dans lequel, pour régler la position de la table, une commande correspondante de la table (14, 16) est effectuée.
